(19) Europäisches Patentamt
European Patent Office
Office européen des brevets

(11) Numéro de publication: **0 183 584**
**B1**

# (12) FASCICULE DE BREVET EUROPÉEN

(45) Date de publication du fascicule du brevet: **06.12.89**

(21) Numéro de dépôt: **85402052.6**

(22) Date de dépôt: **23.10.85**

(51) Int. Cl.⁴: **C 07 D 417/12**, C 07 D 401/12, C 07 D 413/12, A 61 K 31/47, C 07 D 215/56

## (54) Nouveaux esters dérivés de l'acide 4-hydroxy 3-quinoléine carboxylique substitué en 2 par une chaîne alpha-hydroxylée, leur préparation, leur application comme médicaments et les compositions les renfermant.

(30) Priorité: **30.10.84 FR 8416573**

(43) Date de publication de la demande: **04.06.86 Bulletin 86/23**

(45) Mention de la délivrance du brevet: **06.12.89 Bulletin 89/49**

(84) Etats contractants désignés: **AT BE CH DE FR GB IT LI LU NL SE**

(56) Documents cité: **DE-A-3 320 102**

**Le dossier contient des informations techniques présentées postérieurement au dépôt de la demande et ne figurant pas dans le présent fascicule.**

(73) Titulaire: **ROUSSEL- UCLAF, 35, boulevard des Invalides, F-75007 Paris (FR)**

(72) Inventeur: **Clemence, Francois, 2, rue Turgot, F-75009 Paris (FR)**
Inventeur: **Le Martret, Odile, 42, avenue de Versailles, F-75016 Paris (FR)**
Inventeur: **Delevallee, Francoise, 48- 50 avenue de la Dame Blanche, F-94120 Fontenay sous Bois (FR)**

(74) Mandataire: **Bourgouin, André, Département des Brevets ROUSSEL UCLAF B.P. no 9, F-93230 Romainville (FR)**

**EP 0 183 584 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente demande concerne de nouveaux esters dérivés de l'acide 4-hydroxy 3-quinoléine carboxylique substitué en 2 par une chaîne comportant un substituant hydroxy, leur préparation, leur application comme médicaments et les compositions les renfermant.

La demande de brevet allemand DE-A-3 320 102 divulgue les dérivés de l'acide 4-hydroxy 3-quinoléine carboxylique substitués en 2 par une chaîne hydroxylée éventuellement éthérifiée ou estérifiée.

La présente invention concerne les dérivés estérifiés en 2 prévus seulement de manière générale dans la demande de brevet allemand. Les nouveaux esters de l'invention constituent donc une invention de sélection.

La présente invention a ainsi pour objet les composés de formule I:

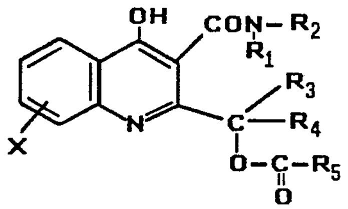

(I)

dans laquelle X en position 5, 6, 7 ou 8 représente un atome d'hydrogène, un atome d'halogène, un radical alcoyle, linéaire ou ramifié, renfermant de 1 à 5 atomes de carbone, un radical alcoxy linéaire ou ramifié, renfermant de 1 à 4 atomes de carbone, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazoyle, imidazoyle, pyrimidyle et tétrazolyle, éventuellement substitués par un radical alcoyle renfermant de 1 à 4 atomes de carbone ou $R_2$ représente un radical phényle, éventuellement substitué par au moins un radical choisi dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène, $R_3$ représente un atome d'hydrogène, un radical alcoyle renfermant de 1 à 4 atomes de carbone ou un radical phényle ou naphtyle, $R_4$ représente un atome d'hydrogène, un radical alcoyle renfermant de 1 à 4 atomes de carbone ou un radical phényle ou naphtyle, $R_5$ représente un radical alcoyle, linéaire ou ramifié, renfermant de 1 à 14 atomes de carbone, éventuellement substitué par un radical amino, alcoylamino ou dialcoylamino dans lequel alcoyle renferme de 1 à 6 atomes de carbone, un radical alcényle renfermant de 2 à 6 atomes de carbone éventuellement substitué par un radical aryle renfermant de 6 à 14 atomes de carbone ou $R_5$ représente un radical aryle renfermant de 6 à 14 atomes de carbone, ou un radical pyridinyle, sous leurs formes racémiques ou optiquement actives ainsi que leurs sels d'addition avec les acides étant entendu que lorsque X représente un radical trifluorométhyle en position 8, $R_1$ représente un atome d'hydrogène, $R_2$ représente un radical 2-thiazolyle, $R_3$ représente un atome d'hydrogène et $R_4$ représente un radical méthyle, $R_5$ ne représente pas un radical méthyle.

- Lorsque X représente un atome d'halogène, il s'agit de préférence d'un atome de chlore.
- Lorsque X représente un radical alcoyle, il s'agit de préférence du radical méthyle, éthyle, n-propyle, n-butyle, n-pentyle, isopropyle ou isobutyle.
- Lorsque X représente un radical alcoxy, il s'agit de préférence du radical méthoxy, éthoxy ou n-propoxy.
- Lorsque $R_1$ représente un radical alcoyle, il s'agit de préférence du radical méthyle ou éthyle.
- Lorsque $R_2$ représente un radical pyridinyle substitué par un radical alcoyle, il s'agit de préférence d'un radical pyridinyle substitué par un radical méthyle ou éthyle.
- Lorsque $R_2$ représente un radical phényle substitué, il s'agit de préférence d'un radical phényle substitué par au moins un radical choisi dans le groupe formé par les radicaux hydroxy, les radicaux méthyle et éthyle, les radicaux méthoxy et éthoxy, le radical trifluorométhyle, le radical nitro et l'atome de chlore.
- Lorsque $R_3$ représente un radical alcoyle, il s'agit de préférence du radical méthyle, éthyle, propyle, isopropyle, butyle ou isobutyle.
- Lorsque $R_4$ représente un radical alcoyle, il s'agit de préférence du radical méthyle, éthyle, propyle, isopropyle, butyle ou isobutyle.
- Lorsque $R_5$ représente un radical alcoyle, il s'agit de préférence du radical méthyle, éthyle, propyle, isopropyle, butyle, isobutyle ou terbutyle.
- Lorsque $R_5$ représente un radical alcényle, il s'agit de préférence d'un radical vinyle, propényle, butényle ou buta-1,3-diényle.
- Lorsque $R_5$ représente un radical aryle ou lorsque $R_5$ représente un radical alcényle substitué par un radical aryle, on entend par aryle un radical phényle ou naphtyle.

Parmi les sels d'addition avec les acides, on peut citer ceux formés avec les acides minéraux tels que les acides chlorhydrique, bromhydrique, sulfurique ou phosphorique ainsi que ceux formés avec les acides sulfoniques tels que les acides alcoyles ou arylsulfoniques, par exemple, l'acide méthanesulfonique ou paratoluènesulfonique.

L'invention a notamment pour objet les composés de formule I pour lesquels X est en position 8, ainsi que

leurs sels d'addition avec les acides et ceux pour lesquels X représente un radical trifluorométhyle ainsi que leurs sels d'addition avec les acides.

L'invention a plus particulièrement pour objet les composés de formule I pour lesquels $R_1$ représente un atome d'hydrogène ainsi que leurs sels d'addition avec les acides et notamment ceux pour lesquels $R_2$ représente le radical thiazolyle ainsi que leurs sels d'addition avec les acides.

Parmi les composés de l'invention, on peut citer notamment les composés pour lesquels $R_3$ représente un atome d'hydrogène ainsi que leurs sels d'addition avec les acides et notamment, parmi ceux-ci, ceux pour lesquels $R_4$ représente un radical éthyle et $R_5$ représente un radical alcoyle renfermant de 1 à 4 atomes de carbone, éventuellement substitué par un radical amino ainsi que leurs sels d'addition avec les acides.

On retient particulièrement les produits cités dans les exemples et tout particulièrement:

- le 2-[1-(1-oxopropoxy)propyl] 4-hydroxy N-(2-thiazolyl) 8-(trifluorométhyl) 3-quinoléine carboxamide, sous formes racémique ou optiquement actives ainsi que ses sels d'addition avec les acides,
- le 2-[1-(1-oxobutoxy) propyl] 4-hydroxy N-(2-thiazolyl) 8-(trifluorométhyl) 3-quinoléine carboxamide, sous formes racémique ou optiquement actives ainsi que ses sels d'addition avec les acides et,
- le 2-[1-(1-oxo 2-aminoéthoxy) propyl] 4-hydroxy N-(2-thiazolyl) 8-(trifluorométhyl) 3-quinoléine carboxamide, sous formes racémiques ou optiquement actives, ainsi que ses sels d'addition avec les acides.

La présente invention a également pour objet un procédé de préparation des produits de formule I, caractérisé en ce que l'on soumet un produit de formule II:

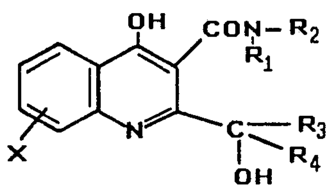

(II)

dans laquelle X, $R_1$, $R_2$, $R_3$ et $R_4$ sont définis comme précédemment, à l'action d'un acide de formule:

$R_5$–COOH

pour obtenir un produit de formule I que l'on soumet, si désiré, à l'action d'un acide pour en former le sel.

Dans des conditions préférentielles d'exécution du procédé ci-dessus:

- on opère en présence de dicyclohexyl carbodiimide et de diméthylamino pyridine, au sein d'un solvant organique;
- dans le cas où l'on souhaite préparer un composé dans lequel $R_5$ représente un radical alcoyle substitué par un radical amino, on utilise un acide dans lequel le radical amino est bloqué et la libération du radical après estérification est effectuée par action d'un acide.
- Lorsque l'on souhaite préparer un produit de formule I optiquement actif, on utilise au départ un alcool que l'on a dédoublé au préalable, par l'intermédiaire d'un acide optiquement actif conduisant à un mélange d'esters que l'on sépare par les moyens classiques, c'est-à-dire par cristallisation ou chromatographie.

Les produits de formule II sont décrits dans EP-A-141 713.

La présente invention a également pour objet un procédé de préparation des produits de formule I dans laquelle $R_1$ représente un atome d'hydrogène, caractérisé en ce que l'on soumet un produit de formule III:

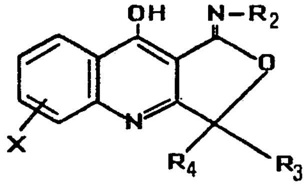

(III)

dans laquelle X, $R_2$, $R_3$ et $R_4$ sont définis comme précédemment, à l'action d'un acide de formule:

$R_5$-COOH

pour obtenir un produit de formule I que l'on soumet, si désiré, à l'action d'un acide pour en former le sel.

Les composés de formule III dans le procédé de la présente invention, correspondent aux produits de formule XII revendiqués dans le brevet belge n° 896 941. Ils peuvent être préparés par le procédé décrit dans ce brevet.

Ils peuvent être également préparés par le procédé suivant, décrit dans la EP-A-141 713.

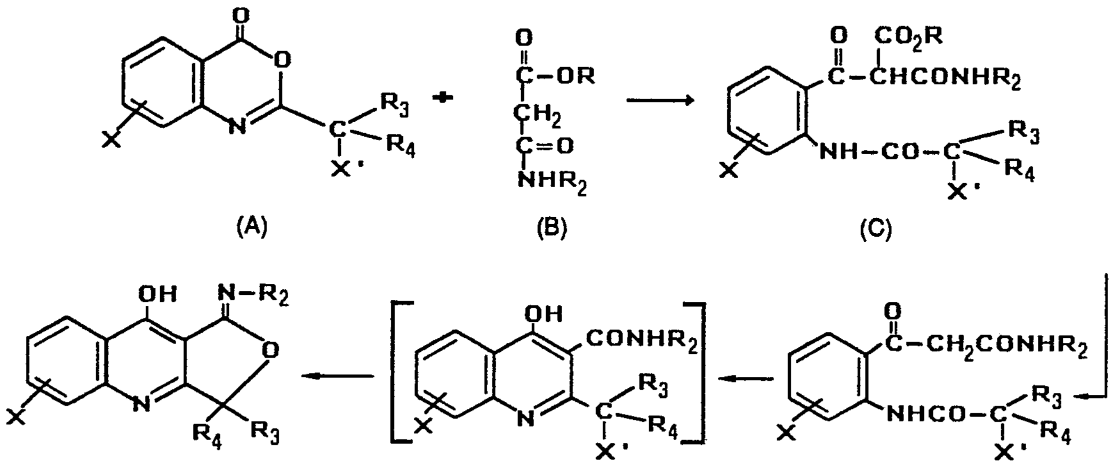

(A) (B) (C)

(III) (D)

Dans les produits de formules A, B, C, D et III, X, $R_2$, $R_3$ et $R_4$ ont les significations précédentes, X' est un atome d'halogène et R représente un radical alcoyle renfermant de 1 à 8 atomes de carbone.

Dans des conditions préférentielles d'exécution du procédé de l'invention, la réaction entre le produit de formule III et l'acide $R_5COOH$ est effectué à une température se situant entre 100 et 150°C, soit, le cas échéant, au reflux de l'acide.

Les composés de la présente demande ainsi que leurs sels d'addition avec les acides, présentent d'intéressantes propriétés pharmacologiques. Ce sont des composés doués de propriétés analgésique et anti-inflammatoire très actifs dans les modèles d'inflammation chronique.

Par rapport aux alcools de formule II décrits dans la EP-A-141 713, les esters de la présente demande et notamment les 3 produits des exemples 2, 6 et 7 présentent une activité anti-arthritique supérieure. En outre, les produits exemples 2 et 6 sont particulièrement bien tolérés au niveau gastrique ainsi que le montre le test figurant ci-après dans la partie expérimentale.

Ces propriétés justifient l'application des composés de formule I en thérapeutique et l'invention a également pour objet, à titre de médicaments, les produits de formule I sous leurs formes racémique ou optiquement actives, ainsi que leurs sels d'addition avec les acides pharmaceutiquement acceptables desdits produits.

L'invention a plus particulièrement pour objet, à titre de médicaments:

- le 2-[1-(1-oxopropoxy) propyl] 4-hydroxy N-(2-thiazolyl) 8-(trifluorométhyl) 3-quinoléine carboxamide, sous formes racémique ou optiquement actives ainsi que ses sels d'addition avec les acides pharmaceutiquement acceptables,
- le 2-[1-(1-oxobutoxy) propyl] 4-hydroxy N-(2-thiazolyl) 8-(trifluorométhyl) 3-quinoléine carboxamide, sous formes racémique ou optiquement actives ainsi que ses sels d'addition avec les acides pharmaceutiquement acceptables,
- le 2-[1-(1-oxo 2-amino éthoxy) propyl] 4-hydroxy N-(2-thiazolyl) 8-(trifluorométhyl) 3-quinoléine carboxamide, sous formes racémique ou optiquement actives, ainsi que ses sels d'addition avec les acides pharmaceutiquement acceptables.

Les médicaments, objet de la présente invention, peuvent être préconisés dans le traitement des maladies inflammatoires dégénératives telles que l'ostéoarthrose, les collagénoses diverses (tendinites), des maladies rhumatismales (polyarthrite rhumatoïde, spondylarthrite enkylosante) ainsi que dans le traitement d'autres maladies de nature auto-immune telles que le lupus érythémateux disséminé, les glomérulonéphrites, la sclérose en plaques.

Les médicaments, objet de l'invention, peuvent également être utilisés dans le traitement des algies musculaires, articulaires ou nerveuses, les douleurs dentaires, les migraines, le zona et également à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

Les exemples donnés ci-après illustrent l'invention sans toutefois la limiter.

**Exemple 1: le 2-(1-acétyloxypropyl) 4-hydroxy N-(2-thiazolyl) 8-trifluorométhyl 3-quinoléine carboxamide.**

On porte au reflux pendant 1 heure 30 une suspension contenant 1 g de 1,3-dihydro 3-éthyl 1-[(2-thiazolyl) imino]5-(trifluorométhyl) furo[3,4-b] quinoléin-9-ol (produit préparé dans le brevet belge n° 896 941 ou comme indiqué dans EP-A-141 713 et 20 $cm^3$ d'acide acétique.

On refroidit la solution obtenue, puis verse sur 20 $cm^3$ d'eau, essore, lave à l'eau et sèche.

On obtient 926 mg de produit que l'on purifie dans l'acétate d'éthyle au reflux. On refroidit, essore et obtient 712 mg de produit attendu fondant à 245°C.

**Exemple 2: 4-hydroxy 2-[1-(1-oxo propoxy) propyl] N-(2-thiazolyl) 8-trifluorométhyl) 3-quinoléine carboxamide.**

On met dans un bain à 100 – 110°C, pendant 2 heures 50, une suspension contenant 3 g de 1,3-dihydro 3-éthyl 1-[(2-thiazolyl) imino]5-(trifluorométhyl) furo[3,4-b] quinoléin-9-ol dans 60 $cm^3$ d'acide propionique.

On refroidit la solution obtenue, puis verse sur 600 $cm^3$ d'eau, essore, lave à l'eau et sèche. On obtient 2,5 g de produit que l'on purifie par recristallisation dans 40 $cm^3$ d'acétate d'éthyle. On refroidit, essore et obtient 1,7 g de produit attendu fondant à 216°C.

**Exemple 3: 2-(1-acétyloxy 2-méthylpropyl) 4-hydroxy N-(2-thiazolyl) 8-trifluorométhyl 3-quinoléine carboxamide.**

**Stade A:** 3-(1-méthyléthyl) 1,3-dihydro 1-[(2-thiazolyl) imino]5-trifluorométhyl furo (3,4-b) quinoléin-9-ol.

1) 2-[(2-chloro 1-oxo 3-méthylbutyl) amino] β-oxo N-(2-thiazolyl) 3-(trifluorométhyl) benzène propanamide.

On opère comme indiqué au stade A de l'exemple 6 du brevet belge N° 896 941 à partir de 10,6 g de N-(2-thiazolyl) acétamide, de 325 $cm^3$ de tétrahydrofuranne, de 106 $cm^3$ de n-butyl lithium dans l'hexane titrant 1,4 M et de 11,4 g de 2-(1-chloro 2-méthylpropyl) 8-(trifluorométhyl) 4H-3,1 benzoxazin 4-one (préparation donnée ci-après) en solution dans 80 $cm^3$ de tétrahydrofuranne.

On obtient 13,8 g de produit attendu fondant à 186°C.

2) 3-(1-méthyléthyl) 1,3-dihydro 1-[(2-thiazolyl) imino] 5-trifluorométhyl furo (3,4-b) quinoléin-9-ol.

On porte au reflux pendant 16 heures 11,65 g de produit obtenu au stade précédent dans 200 $cm^3$ de tétrahydrofuranne et 3,8 g de 4-diméthylaminopyridine. On élimine le tétrahydrofuranne sous pression réduite, ajoute 200 $cm^3$ d'eau au résidu, amène le pH à 1 – 2 avec de l'acide chlorhydrique N. On essore, lave à l'eau, sèche sous pression réduite à 100°C et obtient 10,2 g de produit attendu fondant à 246 – 248°C.

**Préparation de la 2-(1-chloro 2-méthylpropyl) 8-(trifluorométhyl) 4 H-3,1 benzoxazin 4-one.**

On mélange 10,25 g d'acide 2-amino 3-trifluorométhyl benzoïque dans 20 $cm^3$ de toluène et 18,6 g de chlorure de 2-chloro 3-méthyl butanoyle [préparé selon J. Org. Chem. *40*, 3420 (1975)] et opère comme indiqué à la fin de l'exemple 10 du brevet belge N° 896 941 pour la préparation de la 2-(1-chlorobutyl) 8-(trifluorométhyl) 4H-3,1benzoxazin-4-one.

On obtient 11,9 g de produit attendu fondant à 78 – 80°C.

**Stade B:** 2-(1-acétyloxy 2-méthylpropyl) 4-hydroxy N-(2-thiazolyl) 8-trifluorométhyl 3-quinoléine carboxamide.

On porte au reflux, pendant 57 heures, 10 g de produit obtenu au stade A dans 200 $cm^3$ d'acide acétique. On laisse la solution revenir à température ambiante, verse 200 $cm^3$ d'eau, essore, lave à l'eau. On dissout ensuite le produit orangé obtenu dans un mélange de 250 $cm^3$ d'acétate d'éthyle et de 500 $cm^3$ de tétrahydrofuranne, sèche, ajoute 1 g de charbon actif, filtre, concentre à sec sous pression réduite. On obtient 8,3 g de produit que l'on recristallise dans 240 $cm^3$ d'acétate d'éthyle. On filtre à chaud, refroidit, essore, sèche sous pression réduite à 40°C pendant 16 heures. On obtient 5,3 g de produit attendu fondant à 242°C.

**Exemple 4: 4-hydroxy 2-[2-méthyl 1-(1-oxopropoxy) propyl] N-(2-thiazolyl) 8-trifluorométhyl 3-quinoléine carboxamide.**

On porte au reflux pendant 10 heures, une suspension contenant 10 g de produit obtenu au stade A de l'exemple 3 ou obtenu en utilisant le procédé décrit dans EP-A-141 713 dans 200 $cm^3$ d'acide propionique. On laisse revenir à température ambiante puis verse 200 $cm^3$ d'eau, essore et lave à l'eau. On dissout ensuite le résidu obtenu dans un mélange de 300 $cm^3$ d'acétate d'éthyle et de 500 $cm^3$ de tétrahydrofuranne. On sèche, filtre, concentre à sec sous pression réduite. On obtient 8,1 g de produit que l'on recristallise dans 90 $cm^3$ d'acétate d'éthyle. On filtre à chaud, essore, sèche 16 heures sous pression réduite à température ambiance et obtient 5,5 g de produit attendu fondant à 222°C.

**Exemple 5: 4-hydroxy 2-[1-(1-oxo propoxy propyl] N-(2-thiazolyl) 8-trifluorométhyl 3-quinoléine carboxamide.**

Un introduit 1,24 g de dicyclohexyl carbodiimide dans une suspension comprenant 2 g de 4-hydroxy 2-(1-hydroxypropyl) N-(2-thiazolyl) 8-trifluorométhyl 3-quinoléine carboxamide préparé comme au stade C de l'exemple 10 du brevet belge n° 896 941 et 0,4 $cm^3$ d'acide propionique dans 20 $cm^3$ de chlorure de méthylène. On agite 5 minutes, ajoute 0,30 g de diméthylaminopyridine et laisse une heure sous agitation à température ambiante.

On filtre la dicyclohexylurée formée, lave la phase organique à l'acide chlorhydrique N puis avec une solution aqueuse de bicarbonate de soude puis à l'eau, sèche et concentre à sec sous pression réduite.

On reprend le résidu dans 15 $cm^3$ d'éther, essore, sèche sous pression réduite et recueille 2,2 g de produit attendu identique à celui obtenu à l'exemple 2.

F = 216°C.

**Exemple 6: dichlorhydrate de l'amino acétate de 1-[4-hydroxy 3-[(2-thiazolylamino) carbonyl] 8-(trifluorométhyl 2-quinoléinyl] propyle.**

**Stade A:** [(1,1-diméthyl éthoxy carbonyl) amino] acétate de 1-[4hydroxy 3-](2-thiazolylamino) carbonyl] 8-(trifluorométhyl) 2-quinoléinyl] propyle.

On opère comme à l'exemple 5 au départ de 12 g de 4-hydroxy-2-(1hydroxy propyl) N-(2-thiazolyl) 8-trifluorométhyl-3-quinoléine carboxamide et de 5,3 g de N-terbutoxycarbonyl glycine. On obtient 15,8 g de produit attendu.

F = 196 °C.

**Stade B:** dichlorhydrate de l'amino acétate de 1-[4-hydroxy 3-[(2-thiazolylamino) carbonyl] 8-(trifluorométhyl) 2-quinoléinyl] propyle.

On agite 18 heures à température ambiante 14 g de produit préparé comme au stade A, 70 $cm^3$ de chlorure de méthylène et 42 $cm^3$ d'une solution éthanolique d'acide chlorhydrique 5,75 N. On essore le précipité, le lave au chlorure de méthylène, puis à l'éther, le sèche sous pression réduite et obtient 13 g de produit brut que l'on dissout dans 125 $cm^3$ de méthanol puis recristallise par addition de 170 $cm^3$ d'acétate d'éthyle.

On essore, lave à l'acétate d'éthyle, sèche sous pression réduite et recueille 8,4 g de produit attendu. F = 215°C.

**Exemple 7: butanoate de 1-[4-hydroxy 3-[(2-thiazolylamino) carbonyl] 8-(trifluorométhyl) 2-quinoléinyl] propyle.**

On opère comme à l'exemple 5 au départ de 8 g de 4-hydroxy-2-(1-hydroxypropyl)-N-(2-thiazolyl) 8-trifluorométhyl 3-quinoléine carboxamide et de 2 $cm^3$ d'acide butyrique.

On obtient 7,8 g de produit attendu. F = 203°C.

**Exemple 8: 2,2-diméthyl propanoate de 1-[4-hydroxy 3-[(2-thiazolylamino) carbonyl] 8-(trifluorométhyl) 2-quinoléinyl] propyle.**

On opère comme à l'exemple 5 au départ de 5 g de 4-hydroxy-2-(1hydroxy propyl)-N-(2-thiazolyl) 8-trifluorométhyl 3-quinoléine carboxamide et de 1,4 g d'acide pivalique. On obtient 5,15 g de produit attendu. F = 244°C.

**Exemple 9: Dodécanoate de 1-[4-hydroxy 3-[(2-thiazolylamino) carbonyl] 8-(trifluorométhyl) 2-quinoléinyl] propyle.**

On opère comme à l'exemple 5 au départ de 5 g de 4-hydroxy-2-(1hydroxy propyl)-N-(2-thiazoyl) 8-trifluorométhyl 3-quinoléine carboxamide et de 2,8 g d'acide laurique.

On obtient 6 g de produit attendu. F = 158°C.

**Exemple 10: 3-pyridine carboxylate de 1-[4-hydroxy 3-[(2-thiazolylamino) carbonyl] 8-(trifluorométhyl) 2-quinoléinyl] propyle.**

On opère comme à l'exemple 5 au départ de 5 g de 4-hydroxy-2-(1-hydroxy propyl)-N-(2-thiazolyl) 8-trifluorométhyl 3-quinoléine carboxamide et de 1,68 g d'acide nicotinique.

On obtient 4,75 g de produit attendu. F = 200°C.

**Exemple 11: 2-[1-(benzoyloxy) propyl] 4-hydroxy N-(2-thiazolyl) 8-trifluorométhyl 3-quinoléine carboxamide.**

On opère comme à l'exemple 5 au départ de 5 g de 4-hydroxy-2-(1-hydroxy propyl) N-(2-thiazolyl) 8-trifluorométhyl 3-quinoléine carboxamide et de 1,7 g d'acide benzoïque.

On obtient 4,65 g de produit attendu. F = 230°C.

**Exemple 12: 3-phényl 2-propénoate de 1-[4-hydroxy 3-[(2-thiazolylamino) carbonyl] 8-trifluorométhyl 2-quinoléinyl] propyle.**

On opère comme à l'exemple 5 au départ de 5 g de 4-hydroxy 2-(1-hydroxy propyl) N-(2-thiazolyl) 8-trifluorométhyl 3-quinoléine carboxamide et de 2,05 g d'acide cinnamique.

On obtient 4,65 g de produit attendu. F = 210°C.

**Exemple 13: Propanoate de 1-[4-hydroxy 3-[(2-pyridinylamino) carbonyl] 8-trifluorométhyl) 2-quinoléinyl] propyle.**

On porte 5 heures au reflux 6 g de 1,3-dihydro 3-éthyl 2-[(2-pyridinyl) amino 5-trifluorométhyl furo [3,4-b] quinoléin-9-ol en solution dans 120 $cm^3$ d'acide propionique. On refroidit, verse dans 150 $cm^3$ d'eau, essore le précipité, le lave à l'eau et le dissout dans 300 $cm^3$ d'acétate d'éthyle et 50 $cm^3$ de tétrahydrofuranne. On sèche la phase organique, filtre sur charbon actif et concentre à sec sous pression réduite. On recristallise le résidu dans l'acétate d'éthyle, essore, sèche et obtient 3,08 g de produit attendu. F = 204°C.

Préparation du 1,3-dihydro 3-éthyl 2-[(2-pyridinyl) amino 5-trifluorométhyl furo [3,4-b] quinoléin-9-ol.

**Stade A:** 2-[(2-chloro 1-oxobutyl) amino] β-oxo N-(2-pyridinyl) 3-trifluorométhyl benzène propanamide.
On ajoute à 0°C, 314,3 $cm^3$ d'une solution 1,4 M de n-butyllithium dans l'hexane à 30 g de 2-acétylamino pyridine en solution dans 886 $cm^3$ de tétrahydrofuranne. On refroidit à -70°C, ajoute 32 g de 2-(1-chloropropyl) 8-trifluorométhyl 4H-3,1-benzoxazine 4-one préparée selon le procédé décrit à l'exemple 10 du brevet belge n° 896 941 en solution dans 230 $cm^3$ de tétrahydrofuranne. On verse la solution dans 500 $cm^3$ d'acide chlorhydrique 2 N et 600 $cm^3$ d'eau, extrait la phase aqueuse à l'acétate d'éthyle, réunit les phases organiques, sèche et concentre à sec sous pression réduite. On reprend le résidu dans l'éther éthylique, essore, sèche et recueille 9,1 g de produit attendu. F = 137°C.

**Stade B:** 1,3-dihydro 3-éthyl 2-[(2-pyridinyl) amino] 5-trifluorométhyl furo [3,4-b] quinoléin-9-ol.
On chauffe une heure trente minutes au reflux, 27,8 g de produit préparé comme au stade A et 9,46 g de diméthylamino pyridine dans 550 $cm^3$ de dioxane. On refroidit, élimine le solvant sous pression réduite, reprend le résidu dans 300 $cm^3$ d'éther éthylique, essore, dissout le solide dans un mélange chlorure de méthylène – eau (1 – 1). On extrait la phase aqueuse au chlorure de méthylène, réunit les phases organiques, sèche et concentre à sec sous pression réduite. On reprend le résidu à l'éther éthylique, essore et sèche le produit cristallisé.

On obtient 14,4 g de produit attendu. F = 172°C.

**Exemple 14: Acétate de 1-[4-hydroxy 3-[(2-pyridinyl) amino carbonyl] 8-trifluorométhyl 2-quinoléinyl] 2-méthyl propyle.**

On opère comme à l'exemple 13 au départ de 6 g de 1,3-dihydro 3-(1-méthyl éthyl) 1-[(2-pyridinyl) imino] 5-trifluorométhyl furo [3,4-b] quinoléin-9-ol et de 120 $cm^3$ d' acide acétique.

On obtient 3,5 g de produit attendu. F = 230°C.

Préparation du 1,3-dihydro 3-(1-méthyl éthyl) 1-[(2-pyridinyl) imino] 5-trifluorométhyl furo [3,4-b] quinoléin-9-ol.

**Stade A:** 2-[(2-chloro 3-méthyl 1-oxobutyl) amino] β-oxo N-(2-pyridinyl) 3-trifluorométhyl) benzène propanamide.
On opère comme au stade A de la préparation décrite à l'exemple 13 en utilisant au départ 10,16 g de 2-acétylamino-pyridine, 106 $cm^3$ de solution hexanique de n-butyllithium (1,4 M) et de 11,4 g de 2-(1-chloro 2-méthyl propyl) 8-trifluorométhyl 4H-3,1-benzoxazin 4-one dont la préparation est décrite à l'exemple 3.

On obtient 11,8 g de produit attendu. F = 136 – 138°C.

**Stade B:** 1,3-dihydro 3-(1-méthyl éthyl) 1-[(2-pyridinyl) imino] 5-trifluorométhyl furo [3,4-b] quinoléin-9-ol.

On chauffe 7 heures 30 minutes au reflux 10,5 g de produit préparé au stade A, 2,9 g de 4-diméthylamino pyridine dans 100 $cm^3$ de tétrahydrofuranne, refroidit et élimine le solvant sous pression réduite

On reprend le résidu dans 100 $cm^3$ d'eau et 10 $cm^3$ d'acétone, essore, lave à l'eau, sèche à 70°C sous pression réduite et obtient 7,8 g de produit attendu. F = 178 – 180°C.

**Exemple 15: Propanoate de 1-[4-hydroxy 3-[(2-pyridinyl) amino carbonyl] 8-trifluorométhyl 2-quinoléinyl] 2-méthyl propyle.**

On opère comme à l'exemple 13 au départ de 5,9 g de 1,3-dihydro 3-(1-méthyl éthyl) 1-[(2-pyridinyl) imino] 5-trifluorométhyl furo [3,4-b] quinoléin-9-ol préparé comme indiqué à l'exemple 14 et de 120 $cm^3$ d'acide propionique.

On obtient 2,7 g de produit attendu. F = 192°C.

**Exemple 16: 4-hydroxy 2-[1-(1-oxopropoxy) propyl] N-(2-thiazolyl) 8-trifluorométhyl 3-quinoléine carboxamide, isomère A.**

On opère comme à l'exemple 6 à partir de l'isomère A du 4-hydroxy 2-(1-hydroxy propyl) N-(2-thiazolyl) 8-trifluorométhyl 3-quinoléine carboxamide et obtient le produit attendu. F = 187°C.

$\alpha_D$ = –9,5° ± 1,5° (c = 0,7 %, acétone).

Préparation du 4-hydroxy 2-(1-hydroxy propyl) N-(2-thiazolyl) 8-trifluorométhyl 3-quinoléine carboxamide, isomère A.

**Stade A:** α-méthoxy benzène acétate de 1-[4-hydroxy 3-[(2-thiazolyl amino) carbonyl] 8-trifluorométhyl 2-quinoléinyl] propyle, isomères A et B.

On ajoute 13,36 g de dicyclohexylcarbodiimide dans une suspension comprenant 12 g d'acide R(–) méthoxy phényl acétique, 28,7 g de 4-hydroxy 2-(1-hydroxy propyl) N-(2-thiazoyl) 8-trifluorométhyl 3-quinoléine carboxamide (préparé comme à l'exemple 10 du brevet belge n° 896 941) dans 280 $cm^3$ de chlorure de méthylène. On agite, ajoute 4,3 g de diméthylamino pyridine et maintient 2 heures 30 minutes sous agitation. On filtre la dicyclohexylurée formée, lave la phase organique à l'acide chlorhydrique N, puis avec une solution aqueuse saturée de bicarbonate de soude puis à l'eau, sèche et concentre à sec sous pression réduite. On obtient 44,1 g de produit brut que l'on dissout dans 150 $cm^3$ de chlorure de méthylène, élimine l'insoluble par filtration et chromatographie sur silice (éluant : acétate d'éthyle – hexane 1 – 1).

On obtient 15,18 g d'isomère A. F = 172°C.

$\alpha_D$ = –26° ± 3° (c = 0,3 %, chlorure de méthylène) et 14,24 g d'isomère B.F = 194°C.

$\alpha_D$ = –31° ± 1° (c = 1 %, chlorure de méthylène).

**Stade B:** 4-hydroxy 2-(1-hydroxypropyl) N-(2-thiazolyl) 8-trifluorométhyl 3-quinoléine carboxamide, isomère A.

On mélange 13,68 g d'isomère A préparé au stade A dans 70 $cm^3$ de butylamine et abandonne 24 heures à température ambiante. On dilue avec 500 $cm^3$ d'acétate d'éthyle, lave avec 800 $cm^3$ d'acide chlorhydrique 2 N puis à l'eau, sèche et concentre à sec. On obtient 14,7 g de produit brut que l'on recristallise dans l'acétate d'éthyle.

On obtient 3,61 g de produit attendu. F = 180°C.

$\alpha_D$ = –47° ± 2° (c = 0,5%, chloroforme).

**Exemple 17: 4-hydroxy 2-[1-(1-oxopropoxy) propyl] N-(2-thiazolyl) 8-trifluorométhyl 3-quinoléine carboxamide, isomère B.**

On opère comme à l'exemple 6 à partir de l'isomère 8 du 4-hydroxy 2-(1-hydroxy 2-(1-hydroxypropyl) N-(2-thiazolyl) 8-trifluorométhyl 3-quinoléine carboxamide et obtient le produit attendu. F = 187°C.

$\alpha_D$ = +8,5° + 1,5° (c = 0,5 % acétone).

**Préparation du 4-hydroxy 2-(1-hydroxypropyl) N-(2-thiazolyl) 8-trifluorométhyl 3-quinoléine carboxamide, isomère B.**

On opère comme au stade B de la préparation décrite à l'exemple 16 mais au départ de 12,14 g de l'isomère B de l'α-méthoxy benzène acétate de 1-[4-hydroxy 3-[(2-thiazolylamino) carbonyl] 8-trifluorométhyl 2-quinoléinyl] propyle préparé selon le procédé décrit au stade A de la préparation de l'exemple 16 et obtient 3,18 g de produit attendu.

F = 180°C.

$\alpha_D$ = +54° ± 2,5° (c = 0,5 %, chloroforme),

–53,5° ± 2,5° (c = 0,7 %, acétone).

**Exemples de compositions pharmaceutiques.**

Exemple 18: on a préparé des comprimés répondant à la formule suivante:

| | |
|---|---|
| Produit de l'exemple 2 | 50 mg. |
| Excipient q.s. pour un comprimé terminé à | 350 mg. |

(détail de l'excipient: lactose, talc, amidon, stéarate de magnésium).

**Exemple 19: On a préparé des comprimés répondant à la formule suivante:**

| | |
|---|---|
| Produit de l'exemple 7 | 50 mg. |
| Excipient q.s. pour un comprimé terminé à | 350 mg. |

(détail de l'excipient: lactose, talc, amidon, stéarate de magnésium).

**Etude pharmacologique des produits**

**Activité anti-inflammatoire: arthrite chronique à l'adjuvant (traitement préventif).**

L'injection d'adjuvant de type Freund dans une patte postérieure provoque, chez le rat, l'apparition rapide d'une lésion inflammatoire primaire dans cette patte, puis, après un temps de latence de 13 à15 jours, le déclenchement d'une arthrite secondaire affectant notamment l'autre patte postérieure. Le test est pratiqué sur des rats mâles âgés de 42 à 50 jours, qui reçoivent en injection intraplantaire, 0,1 ml d'adjuvant de type "Freund" (suspension dans l'huile de vaseline de 6 mg par ml de mycobacterium butyricum tués).

Les animaux reçoivent le produit étudié, par voie orale, du jour 0 (jour de l'injection de l'adjuvant) jusqu'à la veille du sacrifice, pratiqué le jour 17. Des animaux témoins arthritiques, des animaux témoins normaux ne reçoivent que le véhicule. Les critères d'appréciation de l'activité des substances étudiées sont les augmentations de volume des pattes postérieures injectées (inflammation primaire et secondaire) et non injectées (inflammation secondaire) par rapport au volume moyen des pattes correspondantes de témoins normaux.

On détermine la $DA_{50}$, c'est-à-dire la dose qui diminue de 50 % les augmentations de volume des pattes postérieures des animaux traités par rapport aux animaux témoins.

Les résultats obtenus ont été les suivants:

| Produit de l'exemple | $DA_{50}$ en mg/kg |
|---|---|
| 2 | 0,8 |
| 7 | 1,3 |
| 6 | 1,0 |

**Effet ulcérogène gastrique**

Méthode:

Il a été recherché chez des rats femelles, pesant 120 à 150 g, à la diète hydrique depuis 24 heures au moment du traitement et répartis en blocs randomisés.

Les produits sont administrés per os. Sept heures après, les animaux sont sacrifiés et leurs estomacs, ouverts suivant la grande courbure, sont lavés dans du soluté isotonique de chlorure de sodium et étalés par frottement avec un tampon de coton imbibé de ce même soluté.

L'importance des lésions ulcéreuses, en nombre et en taille est évaluée selon une échelle de 0 à 3 par deux observateurs ignorant les traitements; la notation 1 indique la présence d'un ulcère franc ou de plusieurs ulcérations punctiformes.

Afin de tenir compte également du pourcentage de rats présentant des ulcères (degré d'ulcère supérieur à 0,5, notation attribuée à une hyperhémie ou à des pétéchies souvent rencontrées chez les témoins à jeun), un index d'ulcération est calculé pour chaque groupe selon la formule:

$$\frac{\text{Degré d'ulcère X nombre de rats présentant un ulcère}}{\text{nombre de rats}} \times 100.$$

La dose correspondant à un index d'ulcération de 100 ou DU 100 est déterminée graphiquement (l'index d'ulcération maximum est de 300).

La DU 100 a été trouvée supérieure à 300 mg/kg pour les produits des exemples 2 et 7.

**Revendications**

1. Les composés de formule:

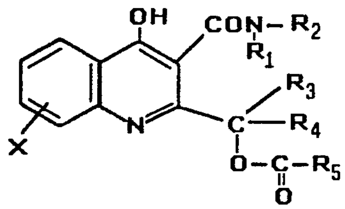

(I)

dans laquelle X en position 5, 6, 7 ou 8 représente un atome d'hydrogène, un atome d'halogène, un radical alcoyle, linéaire ou ramifié renfermant de 1 à 5 atomes de carbone, un radical alcoxy, linéaire ou ramifié renfermant de 1 à 4 atomes de carbone, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R_2$ représente un radical choisi parmi les radicaux thiazolyle, 4,5-dihydrothiazolyle, pyridinyle, oxazolyle, isoxazolyle, imidazolyle, pyrimidyle et tétrazolyle, éventuellement substitués par un radical alcoyle renfermant de 1 à 4 atomes de carbone ou $R_2$ représente un radical phényle, éventuellement substitué par au moins un radical choisi dans le groupe formé par le radical hydroxy, les radicaux alcoyles renfermant de 1 à 4 atomes de carbone, les radicaux alcoxy renfermant de 1 à 4 atomes de carbone, le radical trifluorométhyle, le radical nitro et les atomes d'halogène, $R_3$ représente un atome d'hydrogène, un radical alcoyle renfermant de 1 à 4 atomes de carbone ou un radical phényle ou naphtyle, $R_4$ représente un atome d'hydrogène, un radical alcoyle renfermant de 1 à 4 atomes de carbone ou un radical phényle ou naphtyle, $R_5$ représente un radical alcoyle, linéaire ou ramifié, renfermant de 1 à 14 atomes de carbone, éventuellement substitué par un radical amino, alcoylamino ou dialcoylamino dans lequel alcoyle renferme de 1 à 6 atomes de carbone, un radical alcényle renfermant de 2 à 6 atomes de carbone, éventuellement substitué par un radical aryle renfermant de 6 à 14 atomes de carbone ou $R_5$ représente un radical aryle renfermant de 6 à 14 atomes de carbone ou un radical pyridinyle, sous leurs formes racémiques ou optiquement actives ainsi que leurs sels d'addition avec les acides, étant entendu que lorsque X représente un radical trifluorométhyle en position 8, $R_1$ représente un atome d'hydrogène, $R_2$ représente un radical 2-thiazolyle, $R_3$ représente un atome d'hydrogène et $R_4$ représente un radical méthyle, $R_5$ ne représente pas un radical méthyle.

2. Les composés de formule I telle que décrite à la revendication 1, pour lesquels X est en position 8 ainsi que leurs sels d'addition avec les acides et ceux pour lesquels X représente un radical trifluorométhyle ainsi que leurs sels d'addition avec les acides.

3. Les composés de formule I, telle que décrite à la revendication 1 ou 2, pour lesquels $R_1$ représente un atome d'hydrogène, ainsi que leurs sels d'addition avec les acides.

4. Les composés de formule I telle que définie à la revendication 1, 2 ou 3, pour lesquels $R_2$ représente le radical thiazolyle ainsi que leurs sels d'addition avec les acides.

5. Les composés de formule I telle que définie à l'une quelconque des revendications 1 à 4, pour lesquels $R_3$ représente un atome d'hydrogène, ainsi que leurs sels d'addition avec les acides.

6. Les composés de formule I telle que définie à l'une quelconque des revendications 1 à 5, pour lesquels $R_4$ représente un radical éthyle et $R_5$ représente un radical alcoyle renfermant de 1 à 4 atomes de carbone, éventuellement substitué par un radical amino, ainsi que leurs sels d'addition avec les acides.

7. L'un quelconque des composés de formule (I) dont les noms suivent:

- le 2-[1-(1-oxo propoxy) propyl] 4-hydroxy N-(2-thiazolyl) 8-(trifluorométhyl) 3-quinoléine carboxamide, sous formes racémique ou optiquement actives ainsi que ses sels d'addition avec les acides,
- le 2-[1-(1-oxo butoxy) propyl] 4-hydroxy N-(2-thiazolyl) 8-(trifluorométhyl) 3-quinoléine carboxamide, sous formes racémique ou optiquement actives ainsi que ses sels d'addition avec les acides et,
- le 2-[1-(1-oxo 2-amino éthoxy) propyl] 4-hydroxy N-(2-thiazolyl) 8-(trifluorométhyl) 3-quinoléine carboxamide, sous forme racémique ou optiquement actives, ainsi que ses sels d'addition avec les acides.

8. Procédé de préparation des composés de formule I telle que définie à la revendication 1, caractérisé en ce que l'on soumet un produit de formule II:

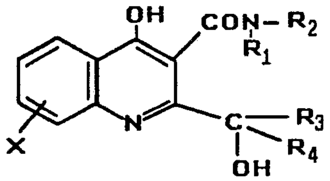

(II)

dans laquelle X, $R_1$, $R_2$, $R_3$ et $R_4$ sont définis comme précédemment, à l'action d'un acide de formule $R_5$–COOH, pour obtenir un produit de formule I que l'on soumet, si désiré, à l'action d'un acide pour en former le sel.

9. Procédé de préparation des composés de formule I telle que définie à la revendication 1, dans laquelle $R_1$ représente un atome d'hydrogène, caractérisé en ce que l'on soumet un produit de formule III:

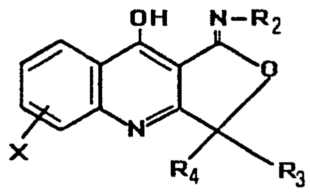

(III)

dans laquelle X, $R_2$, $R_3$ et $R_4$ sont définis comme à la revendication 1, à l'action d'un acide de formule:

$R_5$-COOH

pour obtenir un produit de formule I que l'on soumet, si désiré, à l'action d'un acide pour en former le sel.

10. A titre de médicaments, les composés de formule I telle que définie à la revendication 1, ainsi que leurs sels d'addition avec les acides pharmaceutiquement acceptables.

11. A titre de médicaments, les composés de formule I telle que définie à l'une quelconque des revendications 2 à 6 ainsi que leurs sels d'addition avec les acides pharmaceutiquement acceptables.

12. A titre de médicaments, les composés de formule I tels que définis à la revendication 7 ainsi que leurs sels d'addition avec les acides pharmaceutiquement acceptables.

13. Les compositions pharmaceutiques renfermant comme principe actif au moins un médicament défini à l'une quelconque des revendications 10 à 12.

**Claims**

1. Compounds of formula I

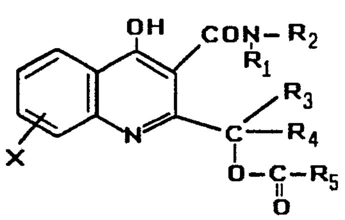

(I)

in which X in position 5, 6, 7 or 8 represents a hydrogen atom, a halogen atom, a linear or branched alkyl radical containing from 1 to 5 carbon atoms, a linear or branched alkoxy radical containing from 1 to 4 carbon atoms, a trifluoromethyl radical, as trifluoromethylthio or trifluoromethoxy radical, $R_1$ represents a hydrogen atom or an alkyl radical containing from 1 to 4 carbon atoms, $R_2$ represents a radical chosen from the following radicals: thiazolyl, 4,5-dihydrothiazolyl, pyridinyl, oxazolyl, isoxazolyl, imidazolyl, pyrimidyl and tetrazolyl, optionally substituted by an alkyl radical containing from 1 to 4 carbon atoms or $R_2$ represents a phenyl radical, optionally substituted by at least one radical chosen from the group formed by the hydroxy radical, alkyl radicals containing from 1 to 4 carbon atoms, alkoxy radicals containing from 1 to 4 carbon atoms, the trifluoromethyl radical, the nitro radical and halogen atoms, $R_3$ represents a hydrogen atom, an alkyl radical containing from 1 to 4 carbon atoms, or a phenyl or naphthyl radical, $R_4$

represents a hydrogen atom, an alkyl radical containing from 1 to 4 carbon atoms or a phenyl or naphthyl radical, $R_5$ represents a linear or branched alkyl radical, containing from 1 to 14 carbon atoms, optionally substituted by an amino, alkylamino or dialkylamino radical in which the alkyl contains from 1 to 6 carbon atoms, an alkenyl radical containing from 2 to 6 carbon atoms optionally substituted by an aryl radical containing from 6 to 14 carbon atoms or $R_5$ represents an aryl radical containing from 6 to 14 carbon atoms or a pyridinyl radical in the racemic or optically active forms as well as their addition salts with acids, it being understood that when X represents a trifluoromethyl radical in position 8, $R_1$ represents a hydrogen atom, $R_2$ represents a 2-thiazolyl radical, $R_3$ represents a hydrogen atom and $R_4$ represents a methyl radical, $R_5$ does not represent a methyl radical.

2. Compounds of formula I as described in claim 1 for which X is in position 8 as well as their addition salts with acids and those for which X represents a trifluoromethyl radical as well as their addition salts with the acids.

3. Compounds of formula I, as described in claims 1 or 2, for which $R_1$ represents a hydrogen atom, as well as their addition salts with acids.
4. Compounds of formula I as defined in claims 1, 2 or 3, for which $R_2$ represents the thiazolyl radical as well as their addition salts with acids.

5. Compounds of formula I as defined in any one of claims 1 to 4, for which $R_3$ represents a hydrogen atom, as well as their addition salts with the acids.

6. Compounds of formula I as defined in any one of claims 1 to 5, for which $R_4$ represents an ethyl radical and $R_5$ represents an alkyl radical containing from 1 to 4 carbon atoms optionally substituted by an amino radical as well as their addition salts with acids.

7. Any one of the compounds of formula I with the following names:

- 2-[1-(1-oxo propoxy) propyl] 4-hydroxy N-(2-thiazolyl) 8-(trifluoromethyl) 3-quinoleine carboxamide, in racemic or optically active form as well as its addition salts with acids,
- 2-[1-(1-oxo butoxy) propyl] 4-hydroxy N-(2-thiazolyl) 8-(trifluoromethyl) 3-quinoleine carboxamide, in racemic or optically active form as well as its addition salts with the acids and,
- 2-[1-(1-oxo 2-amino ethoxy) propyl] 4-hydroxy N-(2-thiazolyl) 8-(trifluoromethyl) 3-quinoleine carboxamide, in racemic or optically active form, as well as its addition salts and acids.

8. Process for the preparation of the compounds of formula I as defined in claim 1, characterized in that a product of formula II:

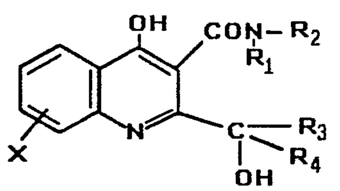

(II)

in which X, $R_1$, $R_2$, $R_3$ and $R_4$ are defined as above, is submitted to the action of an acid of formula $R_5$-COOH, to obtain a product of formula I which is submitted, if desired, to the action of an acid to form a salt.

9. Process for the preparation of the compounds of formula I as defined in claim 1, in which $R_1$ represents a hydrogen atom, characterized in that a product of formula III:

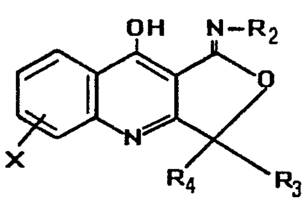

(III)

in which X $R_2$, $R_3$ and $R_4$ are defined as in claim 1, is submitted to the action of an acid of formula:

$R_5$-COOH

to obtain a product of formula I which is submitted, if desired, to the action of an acid to form the salt.

10. As medicaments, compounds of formula I as defined in claim 1 as well as their addition salts with pharmaceutically acceptable acids.

11. As medicaments, compounds of formula I as defined in any one of claims 2 to 6 as well as their addition salts with pharmaceutically acceptable acids.

12. As medicaments, compounds according to claim 7 as well as their addition salts with pharmaceutically acceptable acids.

13. The pharmaceutical compositions containing at least one of the medicaments defined in any one of claims 10 to 12 as active principle.

**Patentansprüche**

1. Verbindungen der Formel I

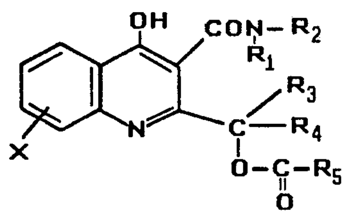

(I)

worin X in 5-, 6-, 7- oder 8-Stellung ein Wasserstoffatom, ein Halogenatom, einen linearen oder verzweigten Alkylrest mit 1 bis 5 Kohlenstoffatomen, eine lineare oder verzweigte Alkoxygruppe mit 1 bis 4 Kohlenstoffatomen, eine Trifluormethylgruppe, eine Trifluormethylthiogruppe oder eine Trifluormethoxygruppe wiedergibt; $R_1$ für ein Wasserstoffatom oder einen Alkylrest mit 1 bis 4 Kohlenstoffatomen steht; $R_2$ einen Rest, ausgewählt unter den Thiazolyl-, 4,5-Dihydrothiazolyl-, Pyridinyl-, Oxazolyl-, Isoxazolyl-, Imidazolyl-, Pyrimidyl- und Tetrazolylresten, die gegebenenfalls durch einen Alkylrest mit 1 bis 4 Kohlenstoffatomen substituiert sind, bedeutet oder $R_2$ einen Phenylrest, der gegebenenfalls durch mindestens einen Rest, ausgewählt unter dem Hydroxyrest, den Alkylresten mit 1 bis 4 Kohlenstoffatomen, den Alkoxyresten mit 1 bis 4 Kohlenstoffatomen, der Trifluormethylgruppe, der Nitrogruppe und den Halogenatomen, substituiert ist, wiedergibt; $R_3$ für ein Wasserstoffatom, einen Alkylrest mit 1 bis 4 Kohlenstoffatomen oder einen Phenyl- oder Naphthylrest steht; $R_4$ ein Wasserstoffatom, eine Alkylgruppe mit 1 bis 4 Kohlenstoffatomen oder eine Phenyl- oder Naphthylgruppe bedeutet; $R_5$ einen linearen oder verzweigten Alkylrest mit 1 bis 14 Kohlenstoffatomen, der gegebenenfalls durch eine Amino-, Alkylamino- oder Dialkylaminogruppe, in der Alkyl 1 bis 6 Kohlenstoffatome umfaßt, substituiert ist, einen Alkenylrest mit 2 bis 6 Kohlenstoffatomen, der gegebenenfalls durch einen Arylrest mit 6 bis 14 Kohlenstoffatomen substituiert ist, bedeutet oder $R_5$ einen Arylrest mit 6 bis 14 Kohlenstoffatomen oder einen Pyridinylrest darstellt, in deren racemischen oder optisch aktiven Formen sowie deren Additionssalze mit Säuren, mit der Maßgabe, daß, wenn X für eine Trifluormethylgruppe in 8-Stellung steht, $R_1$ ein Wasserstoffatom bedeutet, $R_2$ einen 2-Thiazolylrest wiedergibt, $R_3$ für ein Wasserstoffatom steht und $R_4$ eine Methylgruppe bedeutet, $R_5$ keine Methylgruppe bedeutet.

2. Verbindungen der Formel I gemäß Anspruch 1, worin X sich in 8-Stellung befindet, sowie deren Additionssalze mit Säuren, und diejenigen, worin X einen Trifluormethylrest bedeutet, sowie deren Additionssalze mit Säuren.

3. Verbindungen der Formel I gemäß Anspruch 1 oder 2, worin $R_1$ für ein Wasserstoffatom steht, sowie deren Additionssalze mit Säuren.

4. Verbindungen der Formel I gemäß Anspruch 1, 2 oder 3, worin $R_2$ für einen Thiazolylrest steht, sowie deren Additionssalze mit Säuren.

5. Verbindungen der Formel I gemäß einem der Ansprüche 1 bis 4, worin $R_3$ für ein Wasserstoffatom steht, sowie deren Additionssalze mit Säuren.

6. Verbindungen der Formel I gemäß einem der Ansprüche 1 bis 5, worin $R_4$ einen Ethylrest wiedergibt und $R_5$ für einen Alkylrest mit 1 bis 4 Kohlenstoffatomen steht, der gegebenenfalls durch eine Aminogruppe substituiert ist, sowie deren Additionssalze mit Säuren.

7. Eine der Verbindungen der Formel I mit den folgenden Bezeichnungen

- 2-[1-(1-Oxopropoxy)-propyl]-4-hydroxy-N-(2-thiazolyl)-8-(trifluormethyl)-3-chinolin-carboxyamid in racemischer oder optisch aktiver Form sowie dessen Additionssalze mit Säuren,
- 2-[1-(1-Oxobutoxy)-propyl]-4-hydroxy-N-(2-thiazolyl)-8-(trifluormethyl)-3-chinolin-carboxamid in racemischer oder

optisch aktiver Form sowie dessen Additionssalze mit Säuren und

- 2-[-(1-Oxo-2-aminoethoxy)-propyl]-4-hydroxy-N-(2-thiazolyl)-8-(trifluormethyl)-3-chinolin-carboamid in racemischer oder optisch aktiver Form sowie dessen Additionssalze mit Säuren.

8. Verfahren zur Herstellung der Verbindungen der Formel I gemäß Anspruch 1, dadurch gekennzeichnet, daß man ein Produkt der Formel II

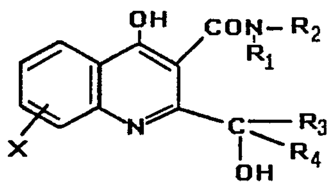

(II)

worin X, $R_1$, $R_2$, $R_3$ und $R_4$ die vorstehende Bedeutung besitzen, der Einwirkung einer Säure der Formel $R_5$-COOH unterzieht, um zu einem Produkt der Formel I zu gelangen, das man gewünschtenfalls der Einwirkung einer Säure unterzieht, um hieraus das Salz zu bilden.

9. Verfahren zur Herstellung der Verbindungen der Formel I gemäß Anspruch 1, worin $R_1$ für ein Wasserstoffatom steht, dadurch gekennzeichnet, daß man ein Produkt der Formel III

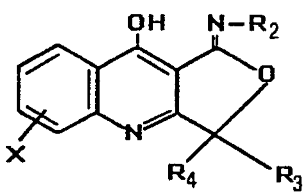

(III)

worin X, $R_2$, $R_3$ und $R_4$ wie in Anspruch 1 definiert sind, der Einwirkung einer Säure der Formel

$R_5$-COOH

unterzieht, um zu einem Produkt der Formel I zu gelangen, das man gewünschtenfalls der Einwirkung einer Säure unterzieht, um hieraus das Salz zu bilden.

10. Als Arzneimittel die Verbindungen der Formel I gemäß Anspruch 1 sowie deren Additionssalze mit pharmazeutisch verträglichen Säuren.

11. Als Arzneimittel die Verbindungen der Formel I gemäß einem der Ansprüche 2 bis 6 sowie deren Additionssalze mit pharmazeutisch verträglichen Säuren.

12. Als Arzneimittel die Verbindungen der Formel I gemäß Anspruch 7 sowie deren Additionssalze mit pharmazeutisch verträglichen Säuren.

13. Pharmazeutische Zusammensetzungen, enthaltend als Wirkstoff zumindest ein Arzneimittel gemäß einem der Ansprüche 10 bis 12.